# EUROPÄISCHE PATENTANMELDUNG

(11) **EP 1 772 168 A1**
(43) Veröffentlichungstag der Anmeldung: **11.04.2007**
(21) Anmeldenummer: 06017212.9
(22) Anmeldetag: 18.08.2006
(51) Int. Cl.: A61Q 5/10, A61K 8/22, A61K 8/38, A61K 8/04, A61K 8/35, A61K 8/42, A61K 8/20, A61K 8/49, A61K 8/66, A61K 8/81

(54) **Verfahren zur oxidativen Färbung keratinhaltiger Fasern**

(30) Priorität: 06.10.2005 DE 102005048183
(71) Anmelder: Henkel Kommanditgesellschaft auf Aktien, 40589 Düsseldorf (DE)
(72) Erfinder: Höffkes, Horst, 40595 Düsseldorf (DE); Bossmann, Britta, 40699 Erkrath (DE)

(57) **Zusammenfassung**

Ein neues Verfahren zur oxidativen Färbung keratinhaltiger Fasern, insbesondere menschlicher Haare, wird beschrieben, in welchem während einer Vorpenetrationsphase die Moleküle mindestens eines Oxidationsfarbstoffvorprodukts und Moleküle mindestens einer, die Farbbildungsreaktion aktivierenden Verbindung in die keratinhaltige Faser eindringen, in dieser Vorpenetrationsphase jedoch kein chemisches Oxidationsmittel auf die keratinhaltige Faser einwirkt und nach Ablauf einer Dauer Z1 der Vorpenetrationsphase in einer darauf folgenden Entwicklungsphase die behandelte Faser mindestens einem chemischen Oxidationsmittel ausgesetzt wird. Das neue Färbeverfahren bewirkt gleichmäßige, intensive Färbungen mit einer hervorragenden Grauabdeckung.

## Beschreibung

Gegenstand der Erfindung ist ein Verfahren zur oxidativen Färbung keratinhaltiger Fasern, insbesondere menschlicher Haare, in welchem in einer Vorpenetrationsphase die Moleküle mindestens eines Oxidationsfarbstoffvorprodukts und Moleküle mindestens einer, die Farbbildungsreaktion aktivierenden Verbindung in die keratinhaltige Faser eindringen, in dieser Vorpenetrationsphase jedoch kein chemisches Oxidationsmittel auf die keratinhaltige Faser einwirkt und nach Ablauf einer Dauer Z1 der Vorpenetrationsphase in einer darauf folgenden Entwicklungsphase die behandelte Faser mindestens einem chemischen Oxidationsmittel ausgesetzt wird. Weiterhin ist eine Verkaufseinheit, enthaltend die zur Durchführung des Verfahrens benötigten Komponenten Gegenstand der Erfindung.

Für das Färben von Keratinfasern, insbesondere von Haaren, Pelzen und Fellen spielen die sogenannten Oxidationsfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklerkomponenten bilden unter dem Einfluß von chemischen Oxidationsmitteln oder von Luftsauerstoff untereinander oder unter Kupplung mit einer oder mehreren Kupplerkomponenten die eigentlichen Farbstoffe aus.

Eine bestimmte Entwicklersubstanz kann durch Kombination mit unterschiedlichen Kupplern auch sehr unterschiedliche Farbnuancen bilden. Trotzdem gelingt es oft nicht, mit Hilfe einer einzigen Entwicklersubstanz zu einer Vielzahl natürlicher Farbnuancen zu kommen. In der Praxis ist daher meist eine Kombination verschiedener Entwicklerkomponenten und Kupplerkomponenten erforderlich, um eine einzige, natürlich wirkende Färbung zu erhalten.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Bei ihrer Anwendung in Haarfärbemitteln sollen sie bereits bei Temperaturen unterhalb 40°C ein gutes Aufziehvermögen auf menschlichem Haar besitzen, wobei keine merklichen Unterschiede zwischen strapaziertem und frisch nachgewachsenem Haar bestehen dürfen (Egalisiervermögen). Sie sollen beständig sein gegen Licht, Wärme und den Einfluß von Haarshampoos und chemischen Reduktionsmitteln, z. B. gegen Dauerwellflüssigkeiten. Schließlich sollen sie die Kopfhaut nicht zu sehr anfärben und vor allem sollen sie in toxikologischer und dermatologischer Hinsicht unbedenklich sein.

Die Luftoxidation läuft meist sehr langsam ab. Daher enthalten die anwendungsbereiten Oxidationsfärbemittel zusätzlich ein chemisches Oxidationsmittel, wie beispielsweise Wasserstoffperoxid, welches die Farbbildungsreaktion durch Oxidation ermöglicht. Allerdings besitzen die auf diese Weise erzielten Färbungen nach wie vor verbesserungswürdige Parameter. Die Steigerung der Farbintensität bei Färbung von ergrautem Haar (Grauabdeckung) bedarf z.B. der Verbesserung.

Zur Steigerung der Farbintensität ist es allgemein üblich, den Färbemitteln sogenannte Aktivatoren bzw. Katalysatoren des Haarfärbeprozesses zuzusetzen. In den Druckschriften Anal.Chem., 1984, 56, 2834-2836, Analyst, 112, Juni 1987, 831-835, Analyst, 115, August 1990, 1103-1107 sowie DE-U1-202 06 612 werden aliphatische und aromatische Aldehyde beschrieben, welche die oxidative Farbbildung der Oxidationsfarbstoffvorprodukte aktivieren sollen.

In den Druckschriften DE-A1-101 18 891, DE-A1-101 18 892 und DE-A1-101 18 893 wird Dihydroxyaceton als potenzieller Aktivator der Farbbildungsreaktion beschrieben.

Im Rahmen der Erfindung wurde festgestellt, dass durch den Einsatz entsprechender Aktivatoren die Grauabdeckung oxidativer Färbungen nicht verbessert werden kann. Werden die Oxidationsfarbstoffvorprodukte gleichzeitig mit dem Aktivator und einem chemischen Oxidationsmittel auf die keratinhaltige Faser aufgebracht und nach einer üblichen Einwirkzeit von 30 Minuten von der Haarfaser entfernt, so ist das sichtbare resultierende Farbergebnis, insbesondere mit Bezug auf die Grauabdeckung, vergleichbar oder schlechter als bei einem Verfahren ohne die Anwendung eines Aktivators.

Zur Verbesserung der Grauabdeckung wird im Rahmen einer Färbung z.B. beim Frisör bekanntermaßen ein Verfahren angewendet, bei dem man in einem ersten Schritt ein Mittel, enthaltend die Oxidationsfarbstoffvorprodukte ohne zusätzliches chemisches Oxidationsmittel, auf die Fasern aufträgt und nach einer Einwirkzeit von üblicherweise 10 Minuten in einem zweiten Schritt ein kosmetisches Mittel mit einem chemischen Oxidationsmittel appliziert. Die Mittel wirken üblicherweise weitere 30 Minuten ein und werden anschließend wieder abgespült. Dieses Verfahren erlaubt im ersten Schritt eine Vorpenetration der Oxidationsfarbstoffvorprodukte in die Faser, bevor das chemische Oxidationsmittel im zweiten Schritt eine Farbstoffbildung bewirkt. Die Einwirkzeiten beider Schritte addieren sich bereits zu einer nachteilig langen Verfahrensdauer. Ein modernes Färbeverfahren sollte möglichst einfach durchführbar sein und eine kurze Durchführungsdauer haben.

Aufgabe der vorliegenden Erfindung ist es daher, ein oxidatives Färbeverfahren für keratinhaltige Fasern bereitzustellen, welches farbintensivere Färbungen sowie insbesondere eine verbesserte Grauabdeckung liefert. Das Verfahren sollte möglichst einfach und mit geringem Zeitaufwand durchführbar sein. Ferner sollte das Verfahren gleichmäßige Färbungen über den Bereich der gesamten Faserlänge ermöglichen. Es wurde nun überraschenderweise gefunden, dass das erfindungsgemäße Verfahren die Aufgabe in hervorragendem Maße löst.

Ein erster Gegenstand der Erfindung ist daher ein Verfahren zur oxidativen Färbung keratinhaltiger Fasern, insbesondere menschlicher Haare, in dem in einer Vorpenetrationsphase
die Moleküle mindestens eines Oxidationsfarbstoffvorprodukts und die Moleküle mindestens einer, die Farbbildungsreaktion aktivierenden Verbindung in die keratinhaltigen Fasern eindringen, in dieser Vorpenetrationsphase jedoch kein chemisches Oxidationsmittel auf die keratinhaltigen Fasern einwirkt
und nach Ablauf einer Dauer Z1 der Vorpenetrationsphase in einer darauf folgenden Entwicklungsphase
auf die behandelten Fasern über eine Dauer Z2 mindestens ein chemisches Oxidationsmittel einwirkt.

Per Definition ist ein Stoff mit einem Schmelzpunkt ≥ 20°C bei einem Druck von 1 atm als fest zu bezeichnen.

Ein erfindungsgemäßes chemisches Oxidationsmittel ist erstens definitionsgemäß in der Lage, die Oxidationsfarbstoffvorprodukte, insbesondere die Entwicklerkomponenten, zu oxidieren und ist zweitens von Luft verschieden.
Als erfindungsgemäßes chemisches Oxidationsmittel dient bevorzugt Wasserstoffperoxid. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon.n H₂O₂ (n ist eine positive ganze Zahl größer 0), Harnstoffperoxid oder Melaminperoxid, eingesetzt.

Die Dauer Z1 beträgt bevorzugt 10 bis 20 Minuten.
Die Dauer Z2 beträgt bevorzugt 10 bis 20 Minuten.

Es ist erfindungsgemäß, zur Initiierung der Vorpenetrationsphase eine Zusammensetzung (die so genannte "Zusammensetzung der Vorpenetrationsphase") auf die Faser zu applizieren, welche in einem kosmetischen Träger mindestens ein Oxidationsfarbstoffvorprodukt und mindestens eine, die Farbbildungsreaktion aktivierende Verbindung enthält. Diese Zusammensetzung wirkt über die Dauer Z1 der Vorpenetrationsphase auf die keratinhaltige Faser ein.

Zwischen der Vorpenetrationsphase und der Entwicklungsphase findet kein Spülschritt statt.

Bevorzugterweise dringen in der Entwicklungsphase die Moleküle des chemischen Oxidationsmittels in die keratinhaltigen Fasern ein.

Die Entwicklungsphase kann erfindungsgemäß auf mindestens zwei Arten eingeleitet werden:
Gemäß Ausführungsform (A) wird zur Einleitung der Entwicklungsphase nach Abschluß der Vorpenetrationsphase eine Zusammensetzung (die sogenannte "Zusammensetzung der Entwicklungsphase"), enthaltend in einem kosmetischen Träger mindestens ein chemisches Oxidationsmittel auf die behandelte Faser appliziert. Gemäß dieser Ausführungsform ist es bevorzugt, dass die Zusammensetzung der Vorpenetrationsphase von chemischen Oxidationsmitteln frei ist. In einer besonders bevorzugten Ausführungsform des Verfahrens der Ausführungsform (A) wird demnach in Schritt
   (i) eine Zusammensetzung der Vorpenetrationsphase, enthaltend in einem kosmetischen Träger eine Kombination aus mindestens einem Oxidationsfarbstoffvorprodukt mit mindestens einer, die Farbbildungsreaktion aktivierenden Verbindung, auf die Fasern aufgebracht,
   nach einer Einwirkzeit mit der Dauer Z1 wird in einem folgenden Schritt
   (ii) eine Zusammensetzung der Entwicklungsphase, enthaltend in einem kosmetischen Träger mindestens ein chemisches Oxidationsmittel auf die Fasern aufgetragen und
   nach einer Einwirkzeit mit der Dauer Z2 werden beide Mittel ausgespült, mit der Maßgabe, daß die Zusammensetzung der Vorpenetrationsphase frei von chemischen Oxidationsmitteln ist.
Die Dauer Z1 und die Dauer Z2 betragen auch in dieser Ausführungsform (A) bevorzugt die zuvor als bevorzugt genannten Zeiträume.
Gemäß Ausführungsform (B) wird zur Einleitung der Vorpenetrationsphase eine Zusammensetzung, enthaltend in einem kosmetischen Träger mindestens ein Oxidationsfarbstoffvorprodukt, mindestens eine, die Farbbildungsreaktion aktivierende Verbindung sowie mindestens ein partikelförmiges, mit einer Sperrschicht beschichtetes, festes (gegebenenfalls geträgertes) chemisches Oxidationsmittel auf die Fasern aufgetragen. Die Einwirkzeit dieser Zusammensetzung beträgt die Summe der Dauer Z1 und der Dauer Z2.
   Bevorzugt wird die oben genannte Zusammensetzung unmittelbar vor der Anwendung aus einer Zusammensetzung, enthaltend mindestens ein Oxidationsfarbstoffvorprodukt und mindestens eine, die Farbbildungsreaktion aktivierende Verbindung und einer (bevorzugt festen) Zusammensetzung, enthaltend mindestens ein partikelförmiges, mit einer Sperrschicht beschichtetes, festes, (gegebenenfalls geträgertes) chemisches Oxidationsmittel, gemischt.
Die Dauer Z1 und die Dauer Z2 betragen auch in dieser Ausführungsform (B) bevorzugt die zuvor als bevorzugt genannten Zeiträume.
Ein Vorteil der Ausführungsform (B) ist, dass der Anwender im Verlauf des erfindungsgemäßen Verfahrens nur diese eine Zusammensetzung auf die Faser aufbringen muß, um dadurch zunächst aktiv die Vorpenetrationsphase und dann später passiv die Entwicklungsphase einzuleiten. Der Übergang von der Vorpenetrationsphase in die Entwicklungsphase läuft mittels einer zeitverzögerten Freisetzung des chemischen Oxidationsmittels automatisch ab. Die Sperrschicht des partikelförmigen, mit einer Sperrschicht beschichteten, festen, (gegebenenfalls geträgerten) chemischen Oxidationsmittels löst sich während der Vorpenetrationsphase langsam in der Zusammensetzung auf, bis nach Ablauf der Dauer Z1 das chemische Oxidationsmittel in Lösung geht, die Entwicklungsphase dadurch eingeleitet und die Färbung über die Dauer Z2 entwickelt wird.
Als partikelförmiges, festes chemisches Oxidationsmittel der Ausführungsform (B) werden bevorzugt Anlagerungsprodukte von Wasserstoffperoxid an anorganische oder organische Verbindungen, insbesondere Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid, 2,4-Diamino-6-phenyl-1,3,5-triazinperhydrat (Benzguanperhydrat) und Melaminperoxid, verwendet. Diese liegen wiederum bevorzugt in Pulverform oder als Granulat vor. Die partikelförmigen, festen chemischen Oxidationsmittel werden gemäß Ausführungsform (B) mit der Sperrschicht, bevorzugt enthaltend mindestens eine Verbindung, ausgewählt aus Lactonen, insbesondere gamma- bzw. delta-Lactone von Hydroxycarbonsäuren (wie beispielsweise gamma-Butyrolacton, D-Gluconsäure-5-lacton, D-Gluconsäure-6-lacton, D-Glucuronolacton oder Pantolacton), Carbonsäureanhydriden, insbesondere zyklische Carbonsäureanhydride (Maleinsäureanhydrid, Phthalsäureanhydrid, Benzoesäureanhydrid oder Bernsteinsäureanhydrid), festen, gesättigten oder ungesättigten, linearen oder verzweigten (C₈- bis C₃₀)-Fettsäuren (wie beispielsweise Stearinsäure, Isostearinsäure einem Gemisch aus Stearin- und Isostearinsäure) oder Homo- oder Copolymeren der Acrylsäure (wie beispielsweise Polyacrylsäure oder Maleinsäure/Acrylsäure-Copolymere) versehen. Die Sperrschicht befindet sich an der Oberfläche der Partikel des besagten festen chemischen Oxidationsmittels in Form eines Coatings. Die Sperrschicht kann beispielsweise durch, gegebenenfalls wiederholtes, Besprühen der partikelförmigen, festen (gegebenenfalls geträgerten) chemischen Oxidationsmittel mit einer Lösung des zuvor beschriebenen Beschichtungsmaterials hergestellt werden. Eine Herstellung nach dem bekannten Wirbelbettverfahren ist ebenfalls möglich.
Die Sperrschicht und das partikelförmige, feste chemische Oxidationsmittel liegen bevorzugt in einem Gewichtsverhältnis von 5:1 bis 1:5, besonders bevorzugt von 3:1 bis 1:3, ganz besonders bevorzugt von 2:1 bis 1:2 in dem beschichteten Partikel vor.
Es ist erfindungsgemäß bevorzugt, die keratinhaltigen Fasern generell nach Beendigung der Entwicklungsphase zu spülen und anschließend zu trocknen.
In einer ersten bevorzugten Ausführungsform werden als Oxidationsfarbstoffvorprodukte mindestens eine Entwicklerkomponente und gegebenenfalls zusätzlich mindestens eine Kupplerkomponente in dem erfindungsgemäßen Verfahren eingesetzt.
Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate verwendet.
Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (Ent1) 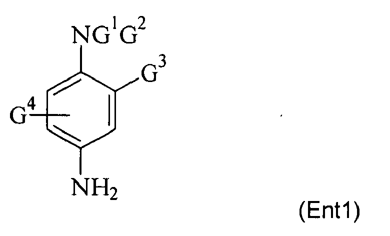
wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (Ent1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (Ent1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (Ent1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in dem Verfahren gemäß der Erfindung verwendet werden können, kann man insbesondere die Verbindungen nennen, die der folgenden Formel (Ent2) entsprechen, sowie ihre physiologisch verträglichen Salze: 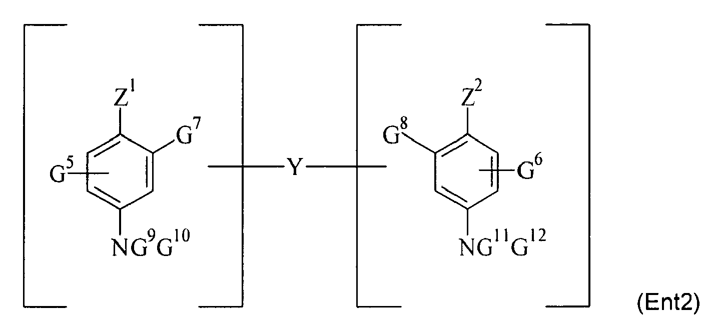 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (Ent2) nur eine Verbrückung Y pro Molekül enthalten und
- die Verbindungen der Formel (Ent2) mindestens eine Aminogruppe enthalten, die mindestens ein Wasserstoffatom trägt.

Die in Formel (Ent2) verwendeten Substituenten sind erfindungsgemäß analog zu den obigen Ausführungen definiert.

Bevorzugte zweikernige Entwicklerkomponenten der Formel (Ent2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (Ent2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (Ent3) 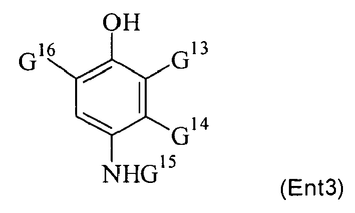 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (Ent3) verwendeten Substituenten sind erfindungsgemäß analog zu den obigen Ausführungen definiert.

Bevorzugte p-Aminophenole der Formel (Ent3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (Ent3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazolopyrimidin-Derivate sind insbesondere die Derivate des Pyrazolo[1,5-a]opyrimidin der folgenden Formel (Ent4) und dessen tautomeren Formen, sofern ein tautomeres Gleichgewicht besteht: 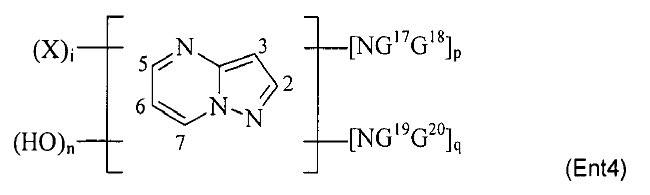 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (Ent4) verwendeten Substituenten sind erfindungsgemäß analog zu den obigen Ausführungen definiert.

Wenn das Pyrazolo[1,5-a]pyrimidin der obenstehenden Formel (Ent4) eine Hydroxygruppe an einer der Positionen 2, 5 oder 7 des Ringsystems enthält, besteht ein tautomeres Gleichgewicht, das zum Beispiel im folgenden Schema dargestellt wird: 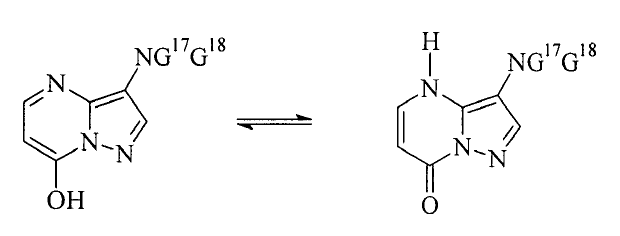 Unter den Pyrazolo[1,5-a]pyrimidinen der obenstehenden Formel (Ent4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]opyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomeres Gleichgewicht vorhanden ist.

Die Pyrazolo[1,5-a]pyrimidine der obenstehenden Formel (Ent4) können wie in der Literatur beschrieben durch Zyklisierung ausgehend von einem Aminopyrazol oder von Hydrazin hergestellt werden.

In einer besonders bevorzugten Ausführungsform finden in dem erfindungsgemäßen Verfahren als Oxidationsfarbstoffvorprodukte neben mindestens einer Entwicklerkomponente zusätzlich mindestens eine Kupplerkomponente Verwendung.

Als Kupplerkomponenten werden in der Regel m-Phenylendiaminderivate, Naphthole, Resorcin und Resorcinderivate, Pyrazolone und m-Aminophenolderivate eingesetzt. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Wenn mindestens eine Kupplerkomponente in dem erfindungsgemäßen Verfahren zum Einsatz kommt, wird als erfindungsgemäß bevorzugte Kupplerkomponente mindestens eine Verbindung aus folgenden Verbindungen verwendet:
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol, o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich kann in der Vorpenetration des erfindungsgemäßen Verfahrens zusätzlich als Vorstufe naturanaloger Farbstoffe mindestens eine Indol- und/oder Indolinverbindung eingesetzt werden, die mindestens eine Hydroxy- oder Aminogruppe, bevorzugt als Substituent am Sechsring, aufweist. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (la), 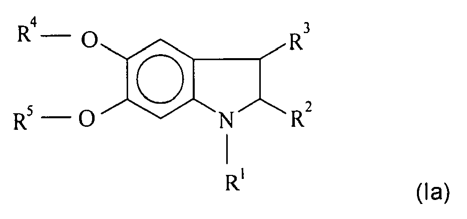 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Erfindungsgemäß besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin,

N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 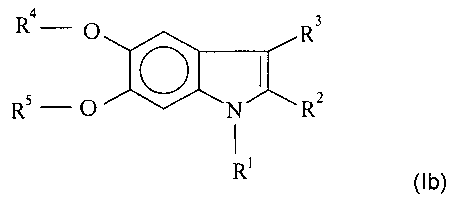 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Erfindungsgemäß besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Zusätzlich kann im Rahmen der Vorpenetrationsphase mindestens ein direktziehender Farbstoff eingesetzt werden. Bevorzugte direktziehende Farbstoffe sind Nitrophenylendiamine, Nitroaminophenole, Azofarbstoffe, Anthrachinone oder Indophenole. Beispiele bevorzugter direktziehender Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol, sowie Mischungen aus mehreren dieser Vertreter

Ferner können die verwendeten direktziehenden Farbstoffe kationische direktziehende Farbstoff sein. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

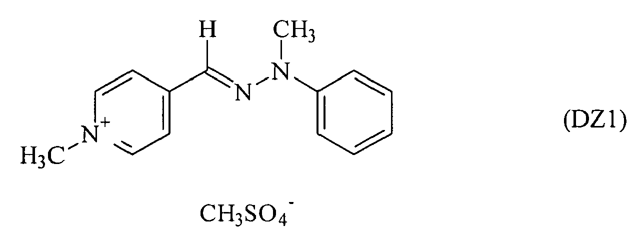

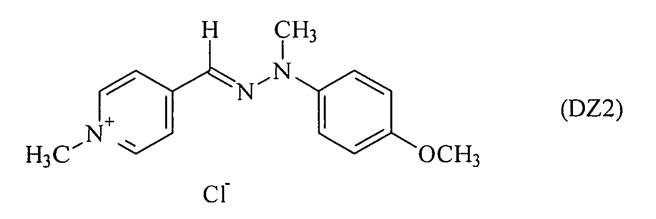

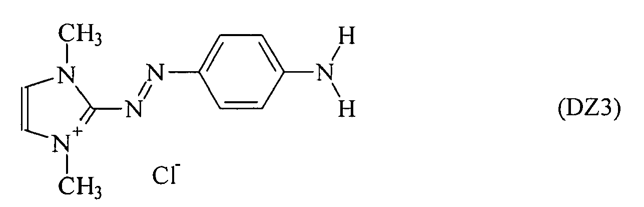

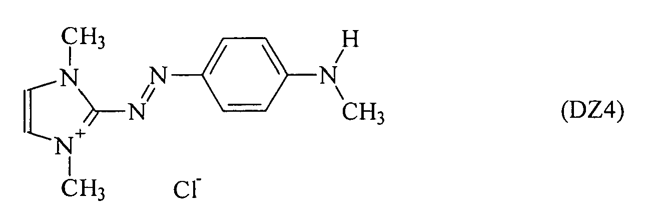

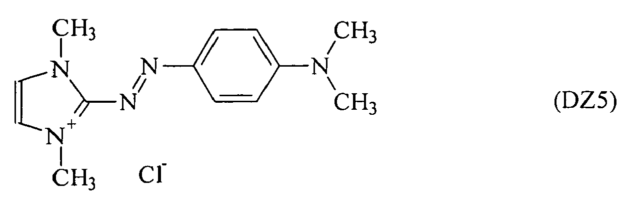

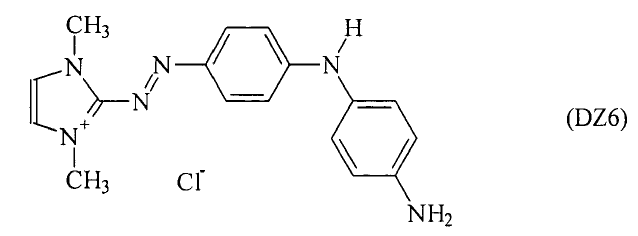

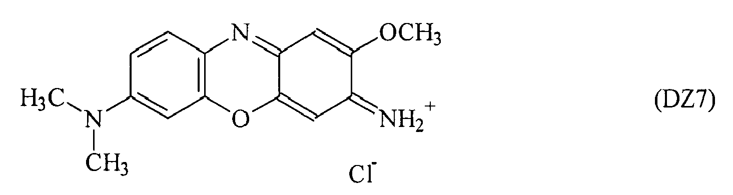

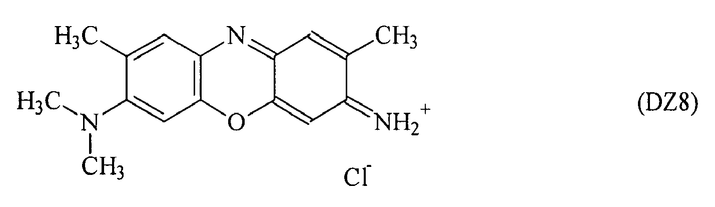

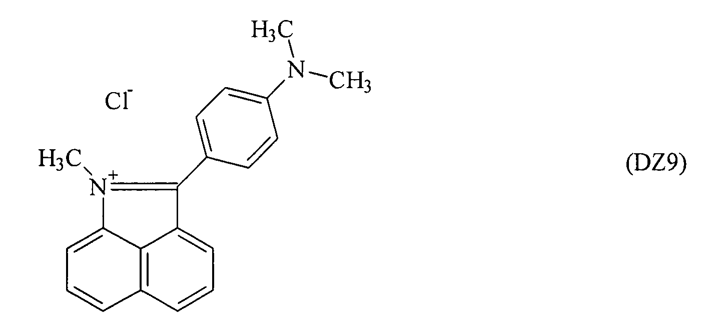

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Weiterhin können während der Vorpenetrationsphase zusätzlich auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, Verwendung finden.

Die Zusammensetzungen zur Einleitung der Vorpenetrationsphase können all die vorgenannten Farbstoffe bzw. Farbstoffvorprodukte, insbesondere in einer Menge von 0,01 bis 20 Gew.-%, bezogen auf das gesamte Mittel, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Zusammensetzungen einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Im Rahmen der Vorpenetrationsphase werden erfindungsgemäß die Oxidationsfarbstoffvorprodukte zusammen mit mindestens einer, die Farbbildungsreaktion aktivierenden Verbindung (Aktivator), angewendet.

Der erfindungsgemäße Aktivator aktiviert oder beschleunigt die in der Entwicklungsphase des erfindungsgemäßen Verfahrens ablaufende Oxidation der Farbstoffvorprodukte durch das chemische Oxidationsmittel. Als ein Aktivator der Farbbildungsreaktion wird erfindungsgemäß bevorzugt mindestens eine Verbindung eingesetzt, die aus mindestens einer Verbindung der Gruppe ausgewählt wird, die gebildet wird aus Metallionen, Iodiden, Chinonen, Enzymen, Imidazol und dessen Derivaten, Carbonatsalzen, Hydrogencarbonatsalzen, Monoalkylcarbonaten und deren Derivate, Silylcarbonaten und deren Derivate, Dihydroxyaceton, aliphatischen Aldehyden und aromatischen Aldehyden. Besonders bevorzugt einsetzbare Aktivatoren sind Metallionen, Hydrogencarbonatsalze, Imidazol und dessen Derivate oder Dihydroxyaceton, sowie Gemische aus diesen Aktivatoren.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die *in situ* geringe Mengen Wasserstoffperoxid erzeugen und auf diese Weise die Oxidation der Farbstoffvorprodukte biokatalytisch aktivieren. Besonders geeignete Aktivatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das Gemisch aus Uricase und Harnsäure bzw. deren Salze ist ein erfindungsgemäß bevorzugt einsetzbares Enzym/Substrat-Gemisch.

Erfindungsgemäß bevorzugt einsetzbare Imidazolverbindungen als Aktivator sind Verbindungen gemäß Formel (II) und/oder dessen physiologisch verträglichen Salze, 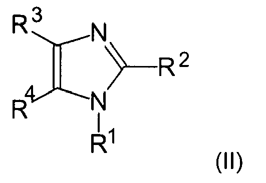 worin
- R¹: steht für ein Wasserstoffatom, eine gegebenenfalls substituierte Arylgruppe oder eine (C₁-C₆)-Alkylgruppe,
- R²: steht für ein Wasserstoffatom, eine Carboxaldehydgruppe, eine (C₁-C₆)-Alkylgruppe oder eine Nitrogruppe,
- R³: steht für ein Wasserstoffatom, eine Carboxy-(C₁-C₆)-alkylgruppe, eine Amino-(C₁-C₆)-alkylgruppe, eine Carboxylgruppe, eine Carboxaldehydgruppe, eine (C₁-C₆)-Alkylgruppe, eine Nitrogruppe, eine 2-Amino-3-hydroxypropylgruppe oder eine Gruppe -CH₂-CH(NH₂)-COOH,
- R⁴: steht für ein Wasserstoffatom, eine Carboxaldehydgruppe oder eine Carboxylgruppe.

Besonders bevorzugt werden die Imidazolverbindungen gemäß Formel II ausgewählt aus einer Gruppe, die gebildet wird, aus Histamin, D-Histidin, L-Histidin, DL-Histidin, D-Histidinol, L-Histidinol, DL-Histidinol, Imidazol, Imidazol-4-essigsäure, Imidazol-4-carbonsäure, Imidazol-4,5-dicarbonsäure, Imidazol-2-carboxaldehyd, Imidazol-4-carboxaldehyd, Imidazol-5-carboxaldehyd, 2-Nitroimidazol, 4-Nitroimidazol, 4-Methylimidazol-5-carboxaldehyd, N-Methylimidazol-2-carboxaldehyd, 4-Methylimidazol, 2-Methylimidazol, N-Methylimidazol, N-(4-Aminophenyl)-imidazol, sowie den physiologisch verträglichen Salzen der vorgenannten Verbindungen.

Als Aktivator erfindungsgemäß bevorzugt einsetzbare Carbonatsalze, bzw. Hydrogencarbonatsalze sind Ammonium-, Alkali- (insbesondere Natrium- und Kalium-), sowie Erdalkali- (insbesondere Calcium-), -carbonatsalze bzw. -hydrogencarbonatsalze.

Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als erfindungsgemäßer Aktivator verwendet werden.

Als Aktivator vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (III),

R-O-C(O)-O-H (III)

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (III) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (III) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in dem erfindungsgemäßen Verfahren Kohlensäuremonoamide eingesetzt werden. Hier ist es erfindungsgemäß bevorzugt, mindestens ein Kohlensäuremonoamid, der Formel (IV) zu verwenden,

R-NH-C(O)-O-H (IV)

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (IV) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Zusammensetzungen sind dadurch gekennzeichnet, daß der Rest R in Formel (IV) ausgewählt ist aus Methyl-, Ethyl-, n-Propyl-, iso-Propyl-, n-Butyl-, iso-Butyl-, *tert*-Butyl- sowie Hydroxymethyl- und Hyd roxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Aktivator vom Typ der Silylcarbonate und deren Derivate werden bevorzugt mindestens ein Silylcarbonat und/oder mindestens ein Silylcarbamat im erfindungsgemäßen Verfahren verwendet. Bevorzugt werden Silylcarbonate gemäß Formel (V) eingesetzt, 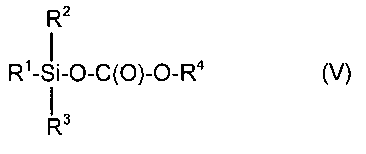 in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (V) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäß verwendete Zusammensetzungen dadurch gekennzeichnet sind, daß die Reste R¹, R² und R³ in Formel (I) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ in der oben genannten Formel (V) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als bevorzugtes Silylcarbamat findet bevorzugt mindestens ein Silylcarbamat der Formel (VI) im erfindungsgemäßen Verfahren Verwendung, 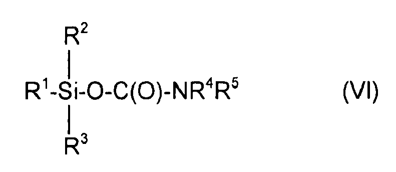 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (VI) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (II) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (VI) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Die Aktivatoren werden bevorzugt in einer Menge von 0.0001 bis 10 Gew.-%, besonders bevorzugt von 0.001 bis 5 Gew.-%, ganz besonders bevorzugt von 0.01 bis 5 Gew.-%, bezogen auf das Gewicht der in der Vorpenetration des erfindungsgemäßen Verfahrens verwendeten Anwendungsmischung, eingesetzt.

Die Zusammensetzung der Vorpenetrationsphase hat bevorzugt einen pH-Wert von 5 bis 11, besonders bevorzugt von 8 bis 10. Dies gilt auch dann bevorzugt, wenn sie zusätzlich gemäß Ausführungsform (B) mindestens ein partikelförmiges, festes, (gegebenenfalls geträgertes) chemisches Oxidationsmittel enthält.

Die Zusammensetzung der Entwicklungsphase gemäß Ausführungsform (A) hat bevorzugt einen pH-Wert von 2 bis 7,5, insbesondere von 2 bis 5.

Die erfindungsgemäße Farbbildungsreaktion findet bevorzugt bei einem pH-Wert von 5 bis 11, insbesondere von 8 bis 10, statt. Dies gilt auch, auch wenn eine Zusammensetzung der Entwicklungsphase mit einem sauren pH-Wert angewendet wird. Der Misch-pH-Wert der beiden Zusammensetzungen auf den Fasern ist in diesem Falle ausschlaggebend.

Die Zusammensetzungen, die im Rahmen des erfindungsgemäßen Verfahrens Anwendung finden, enthalten ihre erfindungsgemäßen Inhaltsstoffe in einem kosmetischen Träger. Solche Träger sind beispielsweise Lösungen, Lotionen, Cremes, Emulsionen, wie z.B. W/O- oder O/W-Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Inhaltsstoffe in eine pulverförmige oder auch tablettenförmige Formulierung zu integrieren, welche vor der Anwendung in einem Lösemittel, wie z.B. Wasser gelöst werden. Die kosmetischen Träger können insbesondere wässrig oder wässrigalkoholisch sein.

Ein wäßriger kosmetischer Träger enthält mindestens 50 Gew.-% Wasser.

Unter wäßrig-alkoholischen kosmetischen Trägern sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die Zusammensetzungen des erfindungsgemäßen Verfahrens können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die kosmetischen Träger der Zusammensetzungen des erfindungsgemäßen Verfahrens können weiterhin alle für solche Zubereitungen bekannten Wirk-, Zusatz- und Hilfsstoffe enthalten. In vielen Fällen enthalten die Zusammensetzungen mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
   Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß können als kationische Tenside insbesondere solche vom Typ der quartären Ammoniumverbindungen, der Esterquats und der Amidoamine eingesetzt werden.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Marken Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Marke Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCl-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die Zusammensetzungen des erfindungsgemäßen Verfahrens weitere Wirk-, Hilfs- und Zusatzstoffe, wie beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen, wie beispielsweise Ammoniak, Ethanolamin oder Alkalihydroxide,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist die Verwendung des Verfahrens des ersten Erfindungsgegenstandes zur Verbesserung der Farbintensität oxidativer Färbungen auf ergrauten keratinhaltigen Fasern, insbesondere ergrautem menschlichen Haar.

Ein dritter Gegenstand der Erfindung ist die Verwendung des Verfahrens des ersten Erfindungsgegenstandes zur Verbesserung der Farbechtheit oxidativer Färbungen auf ergrauten keratinhaltigen Fasern, insbesondere ergrautem menschlichen Haar.

Ein vierter Gegenstand der Erfindung ist eine Verkaufseinheit (Kit), enthaltend als ein oxidatives Färbemittel,
- in einem Container C1 eine Zusammensetzung, enthaltend in einem kosmetischen Träger mindestens ein Oxidationsfarbstoffvorprodukt und mindestens eine, die Farbbildungsreaktion aktivierende Verbindung,
- in einem Container C2 eine Zusammensetzung, enthaltend in einem kosmetischen Träger mindestens ein chemisches Oxidationsmittel.

Eine Verpackungseinheit bildet sich erfindungsgemäß, wenn getrennt konfektionierte Zusammensetzungen durch eine gemeinsame Umverpackung, wie z.B. eine Faltschachtel oder eine Banderole, zusammengeführt werden. Im Sinne der Erfindung gilt es ebenso als Verpackungseinheit, wenn eine Benutzung der getrennt konfektionierten Zusammensetzungen durch Anweisung in der Gebrauchsanleitung beziehungsweise in der Werbung vorgeschlagen wird.

Das chemische Oxidationsmittel der Zusammensetzung in Container C2 wird bevorzugt ausgewählt aus den chemischen Oxidationsmitteln des ersten Erfindungsgegenstandes, sowie aus den partikelförmigen, mit einer Sperrschicht beschichteten, festen, (gegebenenfalls geträgerten) chemischen Oxidationsmitteln des ersten Erfindungsgegenstandes.

Das Kit kann zusätzlich eine Gebrauchsanweisung enthalten, welche die Verwendung des Kits in einem Verfahren gemäß erstem Erfindungsgegenstand beschreibt.

Das Kit kann zusätzlich mindestens einen der folgenden Bestandteile enthalten:
- eine Applikationshilfe, beispielsweise ein Pinsel oder eine Mascarabürste,
- Schutzhandschuhe,
- eine Anrührschale,
- einen Container C3, enthaltend einen Konditioner für keratinhaltige Fasern,
- einen Container C4, enthaltend ein Shampoo.

Ein fünfter Gegenstand der Erfindung ist die Verwendung der Verkaufseinheit des vierten Erfindungsgegenstandes in einem Verfahren des ersten Erfindungsgegenstandes.

Die folgenden Beispiele sollen den Gegenstand der Erfindung näher erläutern:

### Beispiele

### 1.0 Beschichtung eines partikelförmigen, festen chemischen Oxidationsmittels

10 g feinpulvriges Harnstoffperoxid wurden in 6 g eines flüssigen Gemisches aus 1 Teil Isostearinsäure und 2 Teilen Stearinsäure (Erstarrungspunkt der Mischung: 53-55°C) unter intensivem Rühren eingerührt. Nach dem Erkalten wurde das bereits verkapselte Material nochmals auf analoge Weise mit 4 g eines flüssigen Gemisches aus 3 Teilen Isostearinsäure und 1 Teil Stearinsäure (Erstarrungspunkt der Mischung: 40-42°C) verrührt. Nach dem Erstarren wurde das beschichtete Material mit 0,5 g Aerosil 200 der Firma Degussa zur Verminderung der Klebewirkung behandelt. Der Anteil an nicht beschichtetem Material wurde zu 22 % durch Titration der spontan freigesetzten Menge an Wasserstoffperoxid bestimmt.
Eine vergleichend durchgeführte, nur einfache Beschichtung von Harnstoffperoxid mit Isostearinsäure/Stearinsäure 3:1 ergab eine nur unzureichende Beschichtung des Wasserstoffperoxidträgers (nicht beschichteter Anteil 67 %).

### 2.0 Herstellung der Färbemittel

Folgende Basisrezepturen wurden nach üblichen Herstellverfahren unter Verwendung folgender Rohstoffe bereitgestellt:
1 Natriumlaurylethersulfat (27 % Aktivsubstanz; INCl: Sodium Laureth Sulfate) (Hersteller : COGNIS)
2 N,N-Dimethyl-N-(C₈-C₁₈-kokosamidopropyl)ammoniumacetobetain (ca. 30 % Aktivsubstanz; INCl-Bezeichnung: Aqua (Water), Cocamidopropyl Betaine) (Hersteller: COGNIS)
3 Cetylstearylalkohol mit ca. 20 Einheiten Ethylenoxid(INCl-Bezeichnung: Ceteareth-20) (Hersteller: COGNIS)
4 Acrylsäure Copolymer (30% Aktivsubstanz, INCl-Bezeichung: Acrylates Copolymer) (Hersteller: Rohm & Haas)
5 1-Hydroxyethan-1,1-diphosphonsäure (INCl-Bezeichnung: Etidronic Acid, Aqua (Water)) (Hersteller: Solutia)

### 1. Cremebasis

| Rohstoff | Gew.-% |
|---|---|
| Fettalkoholgemisch C₁₂-C₁₈ | 10,5 |
| Texapon^{®} NSO ¹ | 20,0 |
| Dehyton^{®} K ² | 12,5 |
| Eumulgin^{®} B2 ³ | 0,75 |
| ggf. Aktivator | siehe Tabelle 1 |
| Ammoniumsulfat | 0,5 |
| Natriummetabisulfit | 0,4 |
| Entwickler (siehe Tabelle 1) | 1 mMol |
| Kuppler (siehe Tabelle 1) | 1 mMol |
| Wasser | ad 100 |

Der pH-Wert wurde mit wässriger Ammoniaklösung (25 %) auf pH 9,5-10,0 eingestellt.

### 2. Entwicklerlotion

| Rohstoff | Gew.-% |
|---|---|
| Aculyn^{®} 33 ⁴ | 12,0 |
| Wasserstoffperoxid (50 %) | 12,0 |
| Texapon^{®} NSO | 2,0 |
| Turpinal^{®} SL ⁵ | 1,5 |
| Wasser | ad 100 |

Der pH-Wert wurde mit wässriger Ammoniaklösung (25 %) auf pH 3,8-4,2 eingestellt.

### 3.1 Ausfärbungen ohne Vorpenetration

Im Rahmen einer Ausfärbung gemäß Stand der Technik wurde jeweils die farbstoffhaltige Cremebasis aus 2.0 mit den Farbstoffvorprodukten gemäß Tabelle 1 im Verhältnis 1:1 mit der Entwicklerlotion aus 2.0 gemischt. Die Wasserstoffperoxidkonzentration der Mischung beträgt 3 Gew.-%.

Hierbei wurde je eine Ausfärbung mit und eine Ausfärbung ohne Aktivator durchgeführt.

Das frisch gemischte Haarfärbemittel wird im Gewichtsverhältnis von 4 Teilen anwendungsfertigen Färbemittels auf 1 Teil Haar auf das Kerling Euronaturhaar (weiß) aufgetragen. Nach einer Einwirkungszeit von 30 Minuten wurde das Haar gespült und getrocknet.

### 3.2. Ausfärbungen mit Vorpenetration

### 3.2.1 Ausfärbungen gemäß Ausführungsform A der Erfindung

In den Versuchen mit Vorpenetration wurde die farbstoffhaltige Cremebase aus 2.0 (siehe Tabelle 1) mit der Entwicklerlotion aus 2.0, der kein Wasserstoffperoxid zugesetzt wurde, im Gewichtsverhältnis 1:1 gemischt. Hierbei wurde je eine Ausfärbung mit Aktivator (erfindungsgemäß) und zum Vergleich eine Ausfärbung ohne Aktivator (nicht erfindungsgemäß) durchgeführt.

Die oben genannte Mischung wurde im Gewichtsverhältnis von 4 Teilen anwendungsfertigen Färbemittels auf 1 Teil Kerling Euronaturhaar (weiß) zur Vorpenetration aufgetragen. Nach einer Einwirkungszeit (Dauer Z1) der Vorpenetrationsphase von 10 bzw. 15 Minuten (siehe Tabelle 1) wurde der auf dem Haar befindlichen Mischung eine Menge an 50 %-iger Wasserstoffperoxidlösung zugefügt, so dass die Aktivsubstanzmenge an Wasserstoffperoxid 3 Gew.-% berechnet auf das Gewicht der auf dem Haar befindlichen Mischung betrug. Diese Mischung wurde dann 20 bzw. 15 Minuten (siehe Tabelle 1) auf dem Haar belassen. Die Summe der Vorpenetrationsdauer und der Dauer der Entwicklungsphase betrug stets 30 Minuten. Abschließend wurde das Haar gespült und getrocknet.

Die Ergebnisse sind in Tabelle 1 zusammengefasst.

### 3.2.2 Ausfärbung gemäß Ausführungsform B der Erfindung

Die Farbstoffvorprodukt-Kombination Bis-(5-amino-2-hydroxyphenyl)-methan / 2-Amino-3-hydroxypyridin (E2 / K5 aus Tabelle 1) wurde in einem zusätzlichen Experiment mit der Entwicklerlotion aus 2.0 ausgefärbt.

Dabei wurde zunächst einer wasserstoffperoxidfreien Entwicklerlotion aus Punkt 2.0 das in Punkt 1.0 hergestellte, doppelt verkapselte Harnstoffperoxid in einer Menge von 34 g (entspricht einer nominellen Wasserstoffperoxidkonzentration von 6 %) zugemischt. Anschließend wurde die resultierende Mischung mit der Cremebasis im Gewichtsverhältnis 1:1 gemischt. Die Mischung wurde im Gewichtsverhältnis von 4 Teilen anwendungsfertigen Färbemittels auf 1 Teil Kerling Euronaturhaar (weiß) aufgetragen und nach einer Einwirkungszeit von 30 Minuten abgespült. Anschließend wurde das Haar getrocknet.

Die Ergebnisse sind in der folgenden Tabelle 1 aufgeführt.

Die Farbbezeichnungen sind dem Tabellenwerk "Taschenlexikon der Farben" aus dem Musterschmidt Verlag entnommen.

**Tabelle 1:**

| **Entwickler** / **Kuppler** | **Aktivator / Gew.-% in Cremebasis** | **Stand der Technik** | **Stand der Technik** | **Stand der Technik** | **Erfindung** |
|---|---|---|---|---|---|
| | | Standard ohne Aktivator (3.1) | Standard mit Aktivator (3.1) | Vorpenetration ohne Aktivator (3.2.1) | Vorpenetration mit Aktivator (3.2.1)(3.2.2) |
| E1/K1 | Imidazol / 2 % | 10 A5 hellrot | 10 A 5 hellrot | 9 A 5 / A 6 hellrot/rot | 10 A6 / A 7 rot |
| E2 / K2 | (NH₄)HCO₃ / 2 % | 4 C6 / D 6 gold/olivbraun | 4 D 6 olivbraun | 4 D 6 olivbraun | 4 E 6/ F 6 olivbraun ^{A} |
| E2 / K2 | Imidazol / 2 % | 4 C 6 / D 6 gold/olivbraun | 4 D 6 olivbraun ^{E} | 4 D 6 olivbraun ^{D} | 4 E 7 / F 7 olivbraun, ^{B} |
| E2 / K3 | Imidazol / 2 % | 9 D 7 rotbraun | 9 D 5 rotbraun, | 8 D 7 / D 8 rotbraun, ^{D, F} | 9 E 8 / F 8 rotbraun, ^{C, F} |
| E2 / K4 | (NH₄)HCO₃ / 2 % | 5 C 7 braungelb | 5 B 7 / C 7 goldgelb/braungelb | 5 D 8 gelbbraun | 6 D 8 braun |
| E2 / K5 | (NH₄)HCO₃ / 2 % | 5 C 6 / C 7 braunorange/braungelb | 5 C 7 braungelb | 5 D 6 / D 7 hellbraun/goldbraun | 6 D 8 braun ^{G} |
| E2 / K5 | Imidazol / 2 % | 5 C 6 / C 7 braunorange/braungelb | 5 B 6 / C 6 5 grauorange/braunorange | C 8 braungelb | 6 D 8 braun ^{G} |

| | | | | | |
|---|---|---|---|---|---|
| E1 = 2,4,5,6-Tetraaminopyrimidin E2 = Bis-(5-amino-2-hydroxyphenyl)-methan K1 = 2-Methylresorcin K2 = 3-Amino-2-methylamino-6-methoxypyridin K3 = 2-(2,4-Diamino-phenoxy)ethanol K4 = 5-Amino-2-methylphenol K5 = 2-Amino-3-hydroxypyridin ^{A} ¼ Nuance intensiver als 4 D 6 ^{B} ½ Nuance intensiver als 4 D 6 ^{C} 1 Nuance intensiver als 8 D 7 ^{D} ¼ Nuance intensiver als 8 D 7 ^{E} ½ Nuance heller als 8 D 7 ^{F} 15 Minuten Vorpenetration ^{G} Für das verkapselte Harnstoffperoxid als Oxidationsmittel resultieren die gleichen Nuancenausfälle. | | | | | |

In allen Fällen sind die Resultate des erfindungsgemäßen Verfahrens (Vorpenetration mit Aktivator und anschließende Oxidation) ½ - 1 Nuance intensiver als beim Standardfärbeverfahren ohne Vorpenetration und gleichzeitig intensiver als beim Vorpenetrationsverfahren ohne Aktivator.

Wird Wasserstoffperoxid durch Verkapselung des festen Wasserstoffperoxidträgers verzögert freigesetzt (Ausführungsform (B)), so werden die gleichen Intensitäten wie bei der Vorpenetration (Ausführungsform (A)) erzielt.

## Patentansprüche

1. Verfahren zur oxidativen Färbung keratinhaltiger Fasern, insbesondere menschlicher Haare, **dadurch gekennzeichnet, daß** in einer Vorpenetrationsphase
die Moleküle mindestens eines Oxidationsfarbstoffvorprodukts und Moleküle mindestens einer, die Farbbildungsreaktion aktivierenden Verbindung in die keratinhaltigen Fasern eindringen, in dieser Vorpenetrationsphase jedoch kein chemisches Oxidationsmittel auf die keratinhaltige Faser einwirkt
und nach Ablauf einer Dauer Z1 der Vorpenetrationsphase in einer darauf folgenden Entwicklungsphase
auf die behandelten Fasern über eine Dauer Z2 mindestens ein chemisches Oxidationsmittel einwirkt.

2. Verfahren nach Anspruch 1, **dadurch gekennzeichnet, daß** zur Initiierung der Vorpenetrationsphase eine Zusammensetzung, enthaltend in einem kosmetischen Träger mindestens ein Oxidationsfarbstoffvorprodukt und mindestens eine, die Farbbildungsreaktion aktivierende Verbindung, auf die keratinhaltigen Fasern appliziert wird.

3. Verfahren nach Anspruch 2, **dadurch gekennzeichnet, daß** die Zusammensetzung zusätzlich ein partikelförmiges, mit einer Sperrschicht versehenes, festes, chemisches Oxidationsmittel enthält.

4. Verfahren nach Anspruch 3, **dadurch gekennzeichnet, daß** das partikelförmige, mit einer Sperrschicht versehene, feste, chemische Oxidationsmittel mindestens eine Verbindung, ausgewählt aus Anlagerungsprodukten von Wasserstoffperoxid an anorganische oder organische Verbindungen, insbesondere aus der Gruppe Natriumperborat, Natriumpercarbonat, Magnesiumpercarbonat, Natriumpercarbamid, Polyvinylpyrrolidon-n H₂O₂ (n ist eine positive ganze Zahl größer 0), Harnstoffperoxid, 2,4-Diamino-6-phenyl-1,3,5-triazinperhydrat (Benzguanperhydrat) und Melaminperoxid, enthält.

5. Verfahren nach einem der Ansprüche 3 oder 4, **dadurch gekennzeichnet, daß** die Sperrschicht mindestens eine Verbindung, ausgewählt aus ausgewählt aus Lactonen, Carbonsäureanhydriden, festen, gesättigten oder ungesättigten, linearen oder verzweigten (C₈- bis C₃₀)-Fettsäuren oder Homo- oder Copolymeren der Acrylsäure, enthält.

6. Verfahren nach Anspruch 2, **dadurch gekennzeichnet, daß** zur Einleitung der Entwicklungsphase eine Zusammensetzung, enthaltend in einem kosmetischen Träger mindestens ein chemisches Oxidationsmittel, auf die behandelten Fasern appliziert wird, mit der Maßgabe, daß die Zusammensetzung zur Einleitung der Vorpenetrationsphase frei von chemischen Oxidationsmitteln ist.

7. Verfahren nach einem der Ansprüche 1 bis 6, **dadurch gekennzeichnet, daß** nach Beendigung der Dauer Z2 die keratinhaltigen Fasern gespült und anschließend getrocknet werden.

8. Verfahren nach einem der Ansprüche 1 bis 7, **dadurch gekennzeichnet, daß** das chemische Oxidationsmittel Wasserstoffperoxid ist.

9. Verfahren nach einem der Ansprüche 1 bis 8, **dadurch gekennzeichnet, daß** als Oxidationsfarbstoffvorprodukte mindestens eine Entwicklerkomponente und gegebenenfalls zusätzlich mindestens eine Kupplerkomponente eingesetzt werden.

10. Verfahren nach einem der Ansprüche 1 bis 9, **dadurch gekennzeichnet, daß** die die Farbbildungsreaktion aktivierende Verbindung ausgewählt wird aus mindestens einer Verbindung der Gruppe, die gebildet wird aus Metallionen, Iodiden, Chinonen, Enzyme, Imidazol und dessen Derivaten, Carbonatsalzen, Hydrogencarbonatsalzen, Monoalkylcarbonaten und deren Derivate, Silylcarbonaten und deren Derivate, Dihydroxyaceton, aliphatischen Aldehyden und aromatischen Aldehyden.

11. Verwendung des Verfahrens gemäß einem der Ansprüche 1 bis 10 zur Verbesserung der Farbechtheit oxidativer Färbungen auf ergrauten keratinhaltigen Fasern, insbesondere ergrautem menschlichem Haar.

12. Verwendung des Verfahrens gemäß einem der Ansprüche 1 bis 10 zur Verbesserung der Farbechtheit oxidativer Färbungen auf ergrauten keratinhaltigen Fasern, insbesondere ergrautem menschlichem Haar.

13. Verkaufseinheit (Kit), enthaltend als ein oxidatives Färbemittel,
- in einem Container C1 eine Zusammensetzung, enthaltend in einem kosmetischen Träger mindestens ein Oxidationsfarbstoffvorprodukt und mindestens eine, die Farbbildungsreaktion aktivierende Verbindung,
- in einem Container C2 eine Zusammensetzung, enthaltend in einem kosmetischen Träger mindestens ein chemisches Oxidationsmittel.

14. Verwendung der Verkaufseinheit des Anspruchs 13 in einem Verfahren gemäß einem der Ansprüche 1 bis 10.
